# EUROPEAN PATENT APPLICATION

(11) **EP 1 502 606 A1**
(43) Date of publication of application: **02.02.2005**
(21) Application number: 03447200.1
(22) Date of filing: 29.07.2003
(51) Int. Cl.: A61L 9/14

(54) **Device and method for diffusion of a liquid**

(71) Applicant: Polyspray sprl, 1348 Louvain-La-Neuve (BE)
(72) Inventor: Sindayhebura, Daniel, 1020 Brussels (BE); Dumouchel, Christophe, 76000 Rouen (FR)
(74) Representative: Van Malderen, Joelle

(57) **Abstract**

The present invention is related to a device for atomising a liquid (6). The device comprises a liquid reservoir (2), a drive source (1), an ultrasonic atomiser (13) and further a liquid feeding tube (4), connecting the reservoir (2) with the ultrasonic atomiser surface, and an air tube (5), connected between the drive source (1) and the reservoir (2). The drive source (1) is used for air circulation between the air tube (5) and the exterior in order to pressurise or depressurise the reservoir (2), such that a liquid flow can be initiated.

## Description

### Field of the invention

The present invention is related to the diffusion, preferably by ultrasonic atomisation, of sprays of essential oil or of any other liquid having a purifying, bactericidal or deodorising property.

### State of the art

Commonly used systems to diffuse liquid sprays dedicated to air treatment, such as essential oils, are based on an impacting atomisation technique. The liquid feeding system uses an electrical air pump that induces a slight liquid flow in a small tube fixed in a glass bulb, by creating a low-pressure region at its end. As soon as the liquid reaches the tube end, it is hurled by a gas flow against an internal wall of the glass bulb. This impact induces the disintegration of the liquid mass into a cloud of drops of very different sizes. The smallest drops are then transported by the gas flow towards the center of the bulb. This system has several drawbacks among which:
■ it is noisy,
■ it is almost impossible to settle the diffused liquid flow rate,
■ it is fragile,
■ it is not easy to clean when the liquid has to be changed.

The main difficulty related to sprays produced by ultrasonic atomisers consists in finding a liquid feeding system appropriate to any liquid and satisfying the following requirements:
- inversion of the liquid flow direction,
- not noisy when working,
- correct behaviour when working intermittently,
- possible to diffuse very small liquid quantities (a few ml/h),
- full liquid flow rate control,
- simplicity, reliability, compactness (small size),
- easy change of the liquid,
- no maintenance,
- low cost,
- low energy consumption (the whole system is supposed to work with batteries).

Many liquid feeding systems have been proposed for ultrasonic atomisation. A brief overview is given here. In a feeding system that works by gravity there is a high risk of tube blocking when the system is stopped. Furthermore the control of a small liquid flow rate is not easy. With a piston pump there is a risk that when not working, the piston might be stuck on the wall of the pump body. Furthermore, the cost of this technique is high. The main drawback of a membrane pump is a noisy operation. Furthermore, the presence of valves makes the system quite fragile. The system can easily be damaged by liquid drying or polymerisation. Also a gearing pump is easily damageable by liquid drying or polymerisation when the system is not used. A system with a syringe pump is rather expensive. Indeed, the syringe body cannot be in common plastic as to avoid possible chemical reactions with some essential oils and the driving system must be very accurate to ensure small liquid flow rate. Furthermore this system is rather huge.

Peristaltic pumps have been suggested to feed ultrasonic atomisers dedicated to medical treatment (inhaler) (see patent DE-A1-3508560 and EP-B1-0569611). This use is common and consists in sucking up liquid contained in a reservoir and to transport it onto the atomising surface of the atomiser. However, such an application of a peristaltic pump was found inappropriate, as explained in patent US-A-5950619. Due to the flexibility of the circulating tube regularly being squashed high liquid flow rate fluctuations are observed, which are unacceptable in medical treatment.

A classical use of a peristaltic pump to feed an ultrasonic atomiser for essential oil spraying gives rise to other major difficulties:
- Reliability and lifetime : As already mentioned, many diffusing liquids might react with plastic when in contact for a long period of time. This significantly affects the lifetime of the whole system. The solution that consists in choosing special tubing that resists to oil aggression is expensive and is more energy consuming. Besides all this, such tubing becomes less flexible with time.
- High response time : The circulation of the liquid in the pump tube increases the liquid trajectory to the atomising surface of the atomiser. As a consequence, the time delay for the spray emission after switching on the system is rather high. This problem is very critical at very low liquid flow rate.

### Aims of the invention

The present invention aims to provide an apparatus and method for air treatment through the diffusion of a liquid, preferably in the form of sprays, preferably produced by ultrasonic atomisation, that overcome the above-mentioned problems of the state of the art.

### Summary of the invention

The present invention relates to a device for atomising a liquid. The device comprises a reservoir for the liquid, a drive source, an ultrasonic atomiser and further a liquid feeding tube connecting the reservoir with the surface of the ultrasonic atomiser and an air tube connected between the drive source and the reservoir. The drive source is used for air circulation between the air tube and the exterior in order to pressurise or depressurise the reservoir, such that a flow of the liquid can be initiated.

Advantageously, the liquid flow direction in the liquid feeding tube can be inverted.

Preferably the drive source is a peristaltic pump.

In a preferred embodiment the liquid is an essential oil.

Typically the liquid has a purifying, bactericidal or deodorising property.

A second object relates to a method for atomising a liquid contained in a reservoir comprising a liquid feeding tube. The method comprises the steps of :
- providing a drive source for air circulation,
- connecting the drive source to the reservoir by means of an air tube,
- transporting air to the reservoir through the drive source and an air tube at the entrance of the reservoir, thereby inducing a pressure inside the reservoir, resulting in that the liquid is transported through the liquid feeding tube to the surface of an ultrasonic atomiser, and
- atomising the liquid at the surface of the ultrasonic atomiser.

Preferably the drive source in the method is a peristaltic pump.

In a third object the invention relates to a method for purging a liquid feeding tube of a liquid, said liquid feeding tube being in connection with a reservoir of a liquid, comprising the steps of
- providing a liquid flow into the reservoir through the liquid feeding tube,
- transporting air from the reservoir to an inlet of a drive source via an air tube,
- exporting the air via an outlet of a drive source.

### Short description of the drawings

Fig. 1 represents the working principle in case of normal operation.

Fig. 2 represents the working principle when the liquid feeding tube is purged.

Fig. 3 and 4 represent the liquid feeding system with an ultrasonic atomiser.

### Detailed description of the invention

The present invention relates to a new liquid feeding system based on a different use of peristaltic pumps preferably for ultrasonic atomisers for atomising said liquid in a liquid spray 14. A drive source, preferably a peristaltic pump, is used to maintain a pressure slightly greater than the atmospheric pressure in small containers partially filled with essential oil. This pressure is enough to initiate a liquid flow through a feeding tube possibly directed to an ultrasonic atomiser. Furthermore, inverting the rotation of the pump purges advantageously the feeding tube of any liquid.

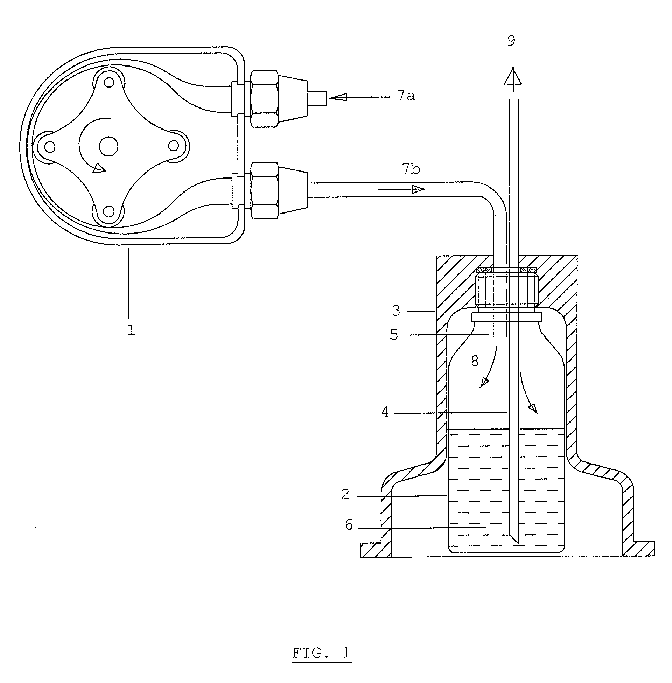

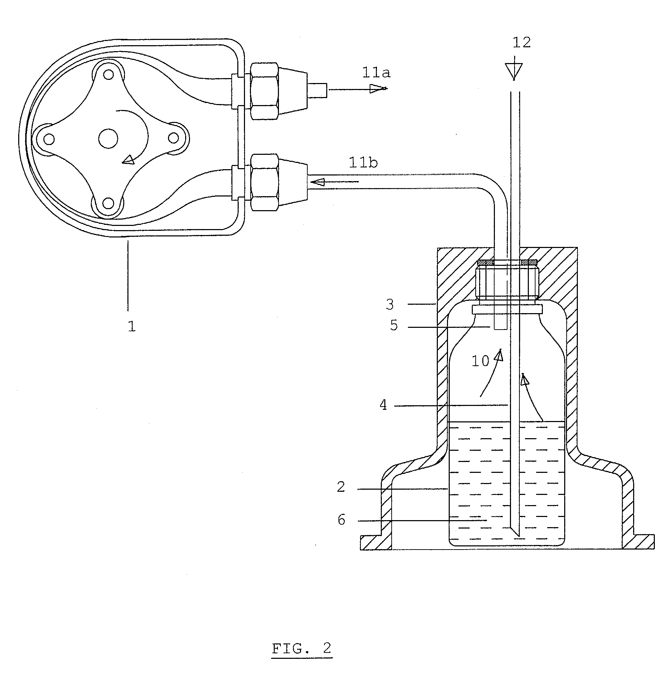

A working principle is presented in the enclosed Figures 1 and 2. Figure 1 is related to the liquid diffusion method step and Figure 2 to the purging method step. In Figure 1 air is brought into a drive source 1 (like e.g. a peristaltic pump) via an air inlet 7a. Via an air outlet 7b the air is transported inside an air tube 5 to a liquid reservoir 2 that is provided with a support 3. Said liquid reservoir is advantageously partially filled with a liquid 6. A pressurisation 8 takes place, which initiates a flow of liquid through a feeding tube 4 to a liquid outlet 9. The scheme of Figure 2 works the other way round. The returning flow enters via liquid inlet 12. In the reservoir a depressurisation 10 is created. Air is now transported from the reservoir to the peristaltic pump 1 via the air tube 5 and an air inlet 11b. Via an air outlet 11a the air is brought to the exterior.

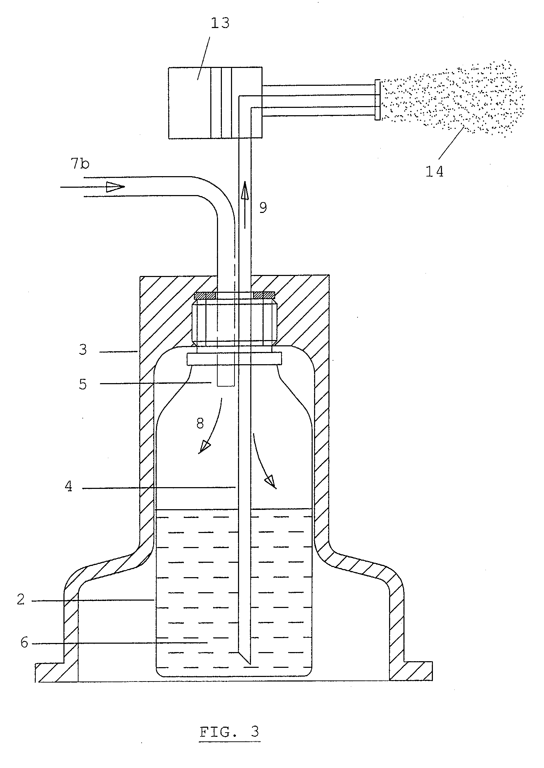

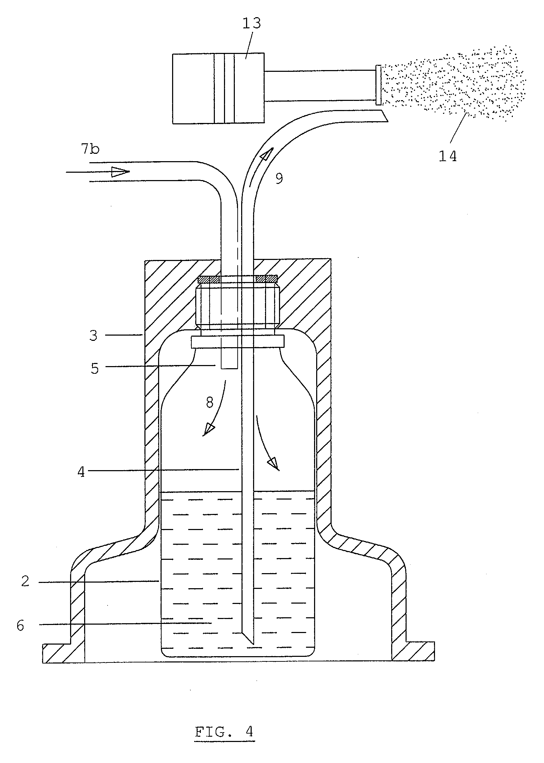

Figures 3 and 4 disclose two specific embodiments of the invention. They show how ultrasonic atomisers 13 can be positioned to minimise the liquid trajectory. The ultrasonic atomiser composed of piezoelectric elements efficiently tied against a mechanical amplifier thanks to an adequate technique. The mechanical amplifier allows increasing the acoustic wave energy generated by the piezoelectric elements up to a sufficient intensity level to initiate and maintain the atomisation process. When the liquid arrives on the atomising surface, it almost instantaneously spreads under vibration effect and forms a film whose thickness is a function of the flow rate. The liquid film is subject to transverse vibrations and a square wave pattern develops on its interface. The liquid feeding can be ensured through a channel (feeding tube 4) made in the mechanical amplifier up to the atomising surface (Fig.3) or through an external tube that ends very near the atomising surface (Fig.4). In similar working conditions as the device of the state of the art the time delay of shortened liquid trajectory systems can be easily divided by three.

The present invention has many advantages:
- The peristaltic pump is used for air circulation only. The lifetime of the pump tube is not a function of the fluid used anymore and is far increased.
- The choice for the feeding tube is not limited to flexible tube anymore. The use of solid feeding tubes that do not interact with essential oil is possible.
- Diminishing the liquid trajectory significantly reduces the time delay of the whole system.
- Finally, this system satisfies all the requirements mentioned before.

## Claims

1. A device for atomising a liquid (6), said device comprising a reservoir (2) for said liquid (6), a drive source (1) and an ultrasonic atomiser (13) **characterised in that** said device further comprises a liquid feeding tube (4) connecting said reservoir (2) with the surface of said ultrasonic atomiser (13) and an air tube (5) connected between said drive source (1) and said reservoir (2), said drive source (1) being used for air circulation between said air tube (5) and the exterior in order to pressurise or depressurise said reservoir, such that a flow of the liquid (2) can be initiated.

2. The device as in claim 1, **characterised in that** the liquid flow direction in said liquid feeding tube (4) can be inverted.

3. The device as in claim 1 or 2, **characterised in that** said drive source (1) is a peristaltic pump.

4. The device as in any of the previous claims, **characterised in that** said liquid is an essential oil.

5. The device as in any of claims 1 to 3, **characterised in that** said liquid has a purifying, bactericidal or deodorising property.

6. A method for atomising a liquid (6) contained in a reservoir (2), said reservoir comprising a liquid feeding tube (4), comprising the steps of :
• providing a drive source (1) for air circulation,
• connecting said drive source (1) to said reservoir (2) by means of an air tube (5),
• transporting air to the reservoir (2) through the drive source (1) and an air tube (5) at the entrance of said reservoir (2), thereby inducing a pressure inside said reservoir (2), resulting in that said liquid is transported through said liquid feeding tube (4) to the surface of an ultrasonic atomiser (13), and
• atomising said liquid at the surface of said ultrasonic atomiser (13).

7. The method of claim 6, **characterised in that** said drive source (1) is a peristaltic pump.

8. A method for purging a liquid feeding tube (4) of a liquid, said liquid feeding tube (4) being in connection with a reservoir (2) of a liquid, comprising the steps of
• providing a liquid flow into said reservoir (2) through said liquid feeding tube (4),
• transporting air from said reservoir (2) to an inlet of a drive source (1) via an air tube (5),
• exporting said air via an outlet of a drive source (1).
